# EUROPÄISCHE PATENTSCHRIFT

(11) **EP 3 424 915 B1**
(45) Veröffentlichungstag und Bekanntmachung des Hinweises auf die Patenterteilung: **30.12.2020**
(21) Anmeldenummer: 18182028.3
(22) Anmeldetag: 05.07.2018
(51) Int. Cl.: C07D 307/46

(54) **VERFAHREN ZUR EXTRAKTION VON 5-HYDROXYMETHYLFURFURAL (5-HMF)**
METHOD FOR THE EXTRACTION OF (5-HYDROXYMETHYLFURFURAL,5-HMF)
PROCÉDÉ D'EXTRACTION DE 5-HYDROXYMÉTHYLFURFURAL (5 HMF)

(30) Priorität: 05.07.2017 EP 17179747
(43) Veröffentlichungstag der Anmeldung: 09.01.2019
(73) Patentinhaber: AVA Biochem AG, 6300 Zug (CH)
(72) Erfinder: MORTATO, Mariangela, 4051 Basel Stadt (CH); BADOUX, Francois, 6314 Unterägeri (CH); MORLEY, Philip, 6330 Cham (CH); DESROCHES, Marie, 8002 Zürich (CH)
(74) Vertreter: Geitz Truckenmüller Lucht Christ

(56) Entgegenhaltungen:
- JP-A- 2013 203 665
- ROBERT-JAN VAN PUTTEN ET AL: "Hydroxymethylfurfural, A Versatile Platform Chemical Made from Renewable Resources", CHEMICAL REVIEWS, Bd. 113, Nr. 3, 11. Februar 2013 (2013-02-11), Seiten 1499-1597, XP055178262, ISSN: 0009-2665, DOI: 10.1021/cr300182k

## Beschreibung

Die Erfindung betrifft Verfahren zur Gewinnung von 5-Hydroxymethylfurfural (5-HMF) aus wässrigen Lösungen.

Das substituierte Furan 5-Hydroxymethylfurfural (HMF) 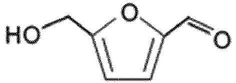 stellt eine wichtige Plattformchemikalie dar und hat als Ausgangsverbindung für zahlreiche Synthesen erhebliche Bedeutung erlangt. 5-HMF lässt sich durch die Behandlung von cellulosehaltiger Biomasse oder von Hexosen unter hydrothermalen Bedingungen erhalten. Die Herstellung von 5-HMF ist bekannt und sieht vor, das Ausgangsmaterial in wässrigem Medium Druck und erhöhter Temperatur auszusetzen. Die damit einhergehende Zersetzung der Hexosen zeichnet sich durch Dehydratisierung und Decarboxylierung sowie Cyclisierung aus. Bei geeigneter Prozessführung handelt es sich bei dem von einer Hydrothermalen Karbonisierung abgeleiteten Verfahren um einen wertvollen Lieferanten für 5-HMF. Die Vielzahl an hierbei sequenziell und parallel ablaufenden chemischen Reaktionen führt zu einer Reihe an Zwischen- und Nebenprodukten, sodass 5-HMF neben weiteren, die Reinheit und Ausbeute vermindernden Verbindungen in einer auch als Prozesswasser bezeichneten wässrigen Lösung vorliegt.

Aufgrund seiner Struktur zeigt 5-HMF eine praktisch unbegrenzte Löslichkeit in Wasser oder in wässrigen Lösungen. Zudem liegt in einer aus dem Herstellungsprozess von 5-HMF stammenden wässrigen Lösung eine Vielzahl weiterer, ebenfalls sehr gut wasserlöslicher Verbindungen oder strukturell sehr eng verwandter Verbindungen mit sehr ähnlichen chemischen und physikalischen Eigenschaften vor. Dies erschwert eine Gewinnung des 5-HMF erheblich.

Aus dem Stand der Technik sind Verfahren bekannt, welche sich der Flüssig-Flüssig-Extraktion mit organischen Lösungsmitteln bedienen, um 5-HMF aus den wässrigen Lösungen der Herstellung zu gewinnen. So beschreibt die JP 2013 203665 eine recht aufwendige Abfolge von mehreren Extraktionsschritten, welche batchweise im Labormaßstab durchgeführt werden. Eine Abfolge von drei Extraktionsschritten liefert zwar 5-HMF mit einer Reinheit von 98,2%, jedoch geht der hohe Reinheitsgrad zu Lasten der Ausbeute.

Zudem besteht der Bedarf an einem Verfahren, welches sich in einem industriellen Maßstab umsetzen lässt.

Vor diesem Hintergrund liegt der vorliegenden Erfindung die Aufgabe zugrunde, ein verbessertes Verfahren zur Gewinnung von 5-HMF aus wässrigen Lösungen der Herstellung bereitzustellen, das neben einer hohen Reinheit des erhaltenen 5-HMF eine Steigerung der Ausbeute ermöglicht und welches im industriellen Maßstab einsetzbar ist.

Dies gelingt durch ein Verfahren gemäß den Merkmalen der Ansprüche 1 und 10. Weitere sinnvolle Ausgestaltungen des Verfahrens können den Unteransprüchen entnommen werden.

Das Verfahren zur Gewinnung von 5-HMF aus einer aus dem Herstellungsprozess von 5-HMF stammenden wässrigen Lösung gemäß Anspruch 1 umfasst die folgenden Schritte:
(A) Kontinuierliche flüssig-flüssig-Extraktion, bei der
   die wässrige Lösung in einer Extraktionskolonne im Gegenstrom mit einem ersten organischen Lösungsmittel, dessen Volumen höchstens die 0,7-fache Menge des Volumens der wässrigen Lösung beträgt, unter Ausbildung einer wässrigen Raffinatphase a1 und einer organischen Phase a2 so in Kontakt gebracht wird, dass in einem ersten Abschnitt der Extraktionskolonne die wässrige Raffinatphase a1 als kontinuierliche Phase und die organische Phase a2 als disperse Phase geführt wird, und dass sich die disperse organische Phase a2 in einem zweiten Abschnitt der Extraktionskolonne vereinigt und eine kontinuierliche organische Phase a2 bildet, und
   die kontinuierliche organische Phase a2 in dem zweiten Abschnitt mit einem polaren Lösungsmittel in Kontakt gebracht wird, welches die Extraktionskolonne im Gegenstrom zu dem organischen Lösungsmittel durchströmt, wobei sich das polare Lösungsmittel in dem ersten Abschnitt mit der wässrigen Raffinatphase a1 vereinigt, und
   die resultierende Raffinatphase a1 der Extraktionskolonne als Raffinat entnommen wird,
(B) Kontinuierliche flüssig-flüssig-Extraktion bei der
   das Raffinat in einer Extraktionskolonne im Gegenstrom mit einem zweiten organischen Lösungsmittel, dessen Volumen mindestens die 2,5-fache Menge des Volumens des Raffinats beträgt, unter Ausbildung einer wässrigen Raffinatphase b1 und einer organischen Phase b2 so in Kontakt gebracht wird, dass in einem Abschnitt der Extraktionskolonne die wässrige Raffinatphase b1 als disperse Phase und die organische Phase b2 als kontinuierliche Phase geführt wird, und
   die organische Phase b2 der Extraktionskolonne als Extrakt entnommen wird.

Überraschenderweise kann durch die Zurverfügungstellung des erfindungsgemäßen Verfahrens aus einer wässrigen Lösung 5-HMF mit einer hohen Ausbeute und gleichzeitig einer hohen Reinheit gewonnen werden.

Im Allgemeinen werden mit dem erfindungsgemäßen Verfahren wässrige Lösungen aufgearbeitet, die neben 5-HMF herstellungsbedingt unter anderem Fructose, Glucose, Zuckerintermediate, Formaldehyd und Carbonsäuren wie Lävulinsäure, Essigsäure und Ameisensäure enthalten. Es wurde gefunden, dass durch die erfindungsgemäße Durchführung des Verfahrens eine effiziente Abtrennung des 5-HMF von diesen Verbindungen ermöglicht wird. Weitere substituierte Furane, wie Furyl-Hydroxymethyl-Keton (FHK) und Furfural, sowie lösliche Furan-Oligomere, welche im Folgenden auch als "unerwünschte Furane" bezeichnet werden und welche ebenfalls in der wässrigen Lösung enthalten sind, weisen aufgrund ihrer Struktur sehr ähnliche chemische und physikalische Eigenschaften auf wie 5-HMF. Es wurde gefunden, dass diese Verbindungen mit dem erfindungsgemäßen Verfahren ebenfalls sehr effizient von 5-HMF abgetrennt werden können.

Unter einer "Flüssig-flüssig-Extraktion" wird erfindungsgemäß eine Extraktion eines in einem flüssigen Lösungsmittel gelösten Stoffes mittels eines zweiten flüssigen Lösungsmittels verstanden.

Unter dem Begriff "wässrige Lösung" wird eine Lösung verstanden, die überwiegend Wasser als Lösungsmittel enthält. In einer 5-HMF enthaltenden wässrigen Lösung können neben dem in der Lösung gelösten 5-HMF weitere Substanzen in der Lösung gelöst oder mit dieser vermischt vorliegen. Die wässrige Lösung kann zudem Substanzen enthalten, die die Extraktion oder die Stabilität des 5-HMF verbessern oder zu bevorzugten Eigenschaften der wässrigen Lösung, wie beispielsweis pH-Wert führen.

Bevorzugt werden in dem erfindungsgemäßen Verfahren wässrige Lösungen aufgearbeitet, welche zwischen 15 und 20 Gew.% 5-HMF aufweisen. Die wässrige Lösung kann jedoch auch geringere oder höhere Mengen an 5-HMF enthalten.

Zur Durchführung des erfindungsgemäßen Verfahrens geeignete organische Lösungsmittel weisen eine ausreichend große Mischungslücke mit der wässrigen Lösung auf. Erstes organisches Lösungsmittel in Schritt (A) und zweites organisches Lösungsmittel in Schritt (B) können identisch oder verschieden sein.

Das erfindungsgemäß in Schritt (A) einsetzbare erste organische Lösungsmittel ist ein organisches Lösungsmittel, mit einem unter den Extraktionsbedingungen hinreichend höheren Verteilungskoeffizienten für die unerwünschten Furane, als für 5-HMF. Halogenierte organische Lösungsmittel wie Chloroform, Chlorbenzol, Trichlorethylen, Tetrachlorkohlenstoff, Fluorchlorkohlenstoffe, Dichlormethan, Tetrachlorethylen oder 1,1,1-Trichlorethan sind geeignet. Neben den reinen Lösungsmitteln können auch Gemische von Lösungsmitteln nützlich sein, sofern sie die hierin für das erste organische Lösungsmittel beschriebenen Eigenschaften aufweisen.

Das erfindungsgemäß in Schritt (B) einsetzbare zweite organische Lösungsmittel weist einen für 5-HMF hinreichend hohen Verteilungskoeffizienten auf. Geeignete Lösungsmittel sind organische Lösungsmittel, die beispielhaft bereits in dem Fachartikel von Blumenthal et al. in ACS Sustainable Chem. Eng. 2016, 4, 228-235 beschrieben sind. Durch eine innige Vermischung von wässriger Lösung und organischem Lösungsmittel wird hierbei der Massetransfer zwischen wässriger Lösung und organischem Lösungsmittel gefördert.

Polare Lösungsmittel sind erfindungsgemäß wässrige Lösungsmittel, inklusive Wasser, oder polare aprotische oder protische organische Lösungsmittel sowie Mischungen von diesen, in denen 5-HMF eine möglichst gute Löslichkeit aufweist.

Bei der Gegenstromextraktion werden wässrige Lösung und organisches Lösungsmittel einer Flüssig-flüssig-Extraktionskolonne an entgegengesetzten Enden zugeführt und beide durchströmen diese kontinuierlich im Gegenstrom. Idealerweise läuft der Trennprozess stufenweise ab, wobei die Raffinatphase (abgereicherte wässrige Lösung) einer Stufe zum Zulauf der nächsten Stufe wird und die Extraktphase (angereichertes Lösungsmittel) einer Stufe zum Extraktionsmittel der vorigen Stufe wird. Hierdurch kann eine hohe Anreicherung von zu extrahierender Substanz im Extraktionsmittel gefördert werden. Dem Fachmann sind verschiedene geeignete Bauformen von Extraktionskolonnen zur Durchführung einer Gegenstromextraktion bekannt. In Frage kommen beispielsweise Sprühkolonnen, Füllkörperkolonnen, Siebbodenkolonnen, pulsierte Füll- oder Siebbodenkolonnen, Drehscheibenkolonnen sowie Mixer-Settler-Batterie-Kolonnen. Üblicherweise umfassen die Extraktionskolonnen ein zylindrisches Rohr, welches als Kolonnenkörper dient. Industriell eingesetzte Extraktionskolonnen können beispielsweise Durchmesser in Bereichen von 0,2 bis 3 m aufweisen. Längen im Bereich von 3 bis 25 m sind ebenfalls nicht selten.

Zur Durchführung des Schritts (A) des erfindungsgemäßen Verfahrens wird beispielsweise in einer vertikal angeordneten Extraktionskolonne am unteren Teil der Extraktionskolonne die 5-HMF enthaltende wässrige Lösung, die Zwischen und Nebenprodukte der Herstellung enthält, kontinuierlich zugeführt. Ein organisches Lösungsmittel mit einer höheren Dichte als die wässrige Lösung wird im oberen Bereich der Extraktionskolonne zugeleitet. Das Volumen des organischen Lösungsmittels beträgt höchstens die 0,7-fache Menge bezogen auf das Volumen der wässrigen Lösung. Unter den erfindungsgemäßen Bedingungen bilden sich ein erster oberer Abschnitt, in dem die wässrige Raffinatphase a1 die kontinuierliche Phase bildet, und ein zweiter unterer Abschnitt mit einer kontinuierlichen organischen Phase a2 im Sumpf der Extraktionskolonne. Derjenige Abschnitt der Extraktionskolonne, in dem sich die disperse organische Phase a2 zu einer kontinuierlichen Phase vereinigt, wird auch Phasengrenze genannt. Im Bereich oberhalb der Phasengrenze wird die organische Phase a2 durch die kontinuierliche wässrige Raffinatphase a1 als disperse, also fein verteilt vorliegende Phase geführt. Bevorzugt wird die wässrige Lösung der Extraktionskolonne oberhalb oder im Bereich der Phasengrenze zugeführt. Eine Zuführung der wässrigen Lösung unterhalb der Phasengrenze ist jedoch ebenfalls möglich.

Die im oberen Abschnitt der Extraktionskolonne als disperse Phase geführte organische Phase a2 reichert sich beim Durchfluss durch die kontinuierliche Raffinatphase a1 mit FHK, Furfural und den löslichen Furan-Oligomeren an, während die kontinuierliche Raffinatphase a1 an diesen Verbindungen verarmt. Aufgrund der hohen strukturellen Ähnlichkeiten zwischen 5-HMF und FHK, Furfural und den löslichen Furan-Oligomeren, wird ebenfalls ein gewisser Anteil des 5-HMF in die organische Phase a2 überführt. Die Co-Extraktion von 5-HMF in die organische Phase a1 resultiert in einer geringeren Ausbeute an 5-HMF in der wässrigen Raffinatphase a1. Sie ist daher unerwünscht. Es wurde jedoch gefunden, dass man den Verlust an 5-HMF auf unter 1 bis 2 Gew.-%, bezogen auf die ursprünglich eingesetzte 5-HMF-Menge, reduzieren kann, wenn man das in die organische Phase a2 extrahierte 5-HMF aus der organischen Phase a2 in die wässrige Raffinatphase a1 rückextrahiert.

Dem unteren Abschnitt der Extraktionskolonne wird ein polares Lösungsmittel mit einer geringeren Dichte als das organische Lösungsmittel zugeführt. Bevorzugt wird das polare Lösungsmittel der Extraktionskolonne in einem Bereich zugeführt, der stromabwärts der Zuführung der wässrigen Lösung liegt - bezogen auf die Strömungsrichtung der organischen Phase a2. Das polare Lösungsmittel durchströmt die Extraktionskolonne aufgrund des Dichteunterschieds im Gegenstrom zu dem organischen Lösungsmittel.

Unterhalb der Phasengrenze wird die kontinuierliche organische Phase a2 mit dem polaren Lösungsmittel durchmischt. Dabei wird das polare Lösungsmittel mit 5-HMF angereichert, während die organische Phase a2 an 5-HMF verarmt. Im oberen Abschnitt der Extraktionskolonne vereinigt sich das mit 5-HMF beladene polare Lösungsmittel mit der wässrigen Raffinatphase a1. Die resultierende wässrige Raffinatphase a1 wird der Extraktionskolonne als Raffinat 1 im oberen Bereich, insbesondere am Kopf der Extraktionskolonne entnommen.

Bei der Flüssig-Flüssig-Extraktion in Schritt (A) werden eine im Wesentlichen von FHK, Furfural sowie löslichen Furan-Oligomeren freie wässrige Raffinatphase a1 und eine mit FHK, Furfural sowie löslichen Furan-Oligomeren beladene organische Phase a2 erhalten. "Im Wesentlichen frei von" bedeutet, dass der Anteil der Verbindungen etwa bis 1,2 Gew.-% relativ zu dem 5-HMF ausmacht. Die aus Schritt (A) resultierende wässrige Raffinatphase a1 enthält gleichzeitig über 98 Gew.-% des ursprünglich eingesetzten 5-HMF.

Zur Durchführung des Schritts (B) des erfindungsgemäßen Verfahrens wird beispielsweise in einer vertikal angeordneten Extraktionskolonne am unteren Teil der Extraktionskolonne das Raffinat der ersten Extraktionskolonne zugeführt. Ein organisches Lösungsmittel mit einer höheren Dichte als das Raffinat wird im oberen Bereich der Extraktionskolonne zugeleitet. Das Volumen des organischen Lösungsmittels beträgt zumindest die 2,5-fache Menge bezogen auf das Volumen des Raffinats. Unter den erfindungsgemäßen Bedingungen bilden sich ein erster oberer Abschnitt, insbesondere am Kopf der Extraktionskolonne, in dem die wässrige Raffinatphase b1 die kontinuierliche Phase bildet, und ein zweiter unterer Abschnitt mit einer kontinuierlichen organischen Phase b2 im unteren Bereich der Extraktionskolonne. Der Abschnitt, in dem sich die disperse wässrige Raffinatphase b1 zu einer kontinuierlichen Phase vereinigt, stellt die Phasengrenze der Extraktionskolonne in Schritt (B) dar. Im Bereich unterhalb der Phasengrenze wird die wässrige Raffinatphase b1 durch die kontinuierliche organische Phase b2 als disperse Phase geführt. Das organische Lösungsmittel kann der Extraktionskolonne unterhalb oder im Bereich der Phasengrenze zugeführt werden. Eine Zuleitung des organischen Lösungsmittels oberhalb der Phasengrenze ist ebenfalls möglich.

Die im unteren Abschnitt der Extraktionskolonne als kontinuierliche Phase geführte organische Phase b2 reichert sich mit 5-HMF an, während die disperse Raffinatphase b1 an 5-HMF verarmt. In der wässrigen Raffinatphase b1 verbleiben die Zwischen- und Nebenprodukte der 5-HMF Herstellung mit einer geringeren Löslichkeit im organischen Lösungsmittel. Die organische Phase b2 kann im unteren Bereich der Extraktionskolonne, insbesondere am Sumpf, als Extrakt entnommen werden.

Im Schritt (B) ist es damit möglich, 5-HMF von Fructose, Glucose, Zuckerintermediaten und Carbonsäuren wie Lävulinsäure, Essigsäure und Ameisensäure abzutrennen. Es kann somit eine wässrige Raffinatphase b1 enthaltend Fructose, Glucose, Zuckerintermediate und Carbonsäuren erhalten werden.

Bei der Durchführung des erfindungsgemäßen Verfahrens wird in Schritt (B) eine mit 5-HMF beladene organische Phase b2 abgetrennt, in der 5-HMF mit einer Reinheit von 97% bis 99%, vorliegt. Die organische Phase b2 enthält gleichzeitig über 95 Gew.-% des ursprünglich eingesetzten 5-HMF.

Nach einer vorteilhaften Ausgestaltung des Verfahrens ist das erste organische Lösungsmittel in Schritt (A) ausgewählt aus
- den halogenierten organischen Lösungsmitteln, insbesondere Chloroform, Chlorbenzol, Trichlorethylen, Tetrachlorkohlenstoff, Fluorchlorkohlenstoffe, Dichlormethan, Tetrachlorethylen oder 1,1,1-Trichlorethan, weiter insbesondere den halogenierten Kohlenwasserstoffen Chloroform, Trichlorethylen, Tetrachlorkohlenstoff, Fluorchlorkohlenstoffe, Dichlormethan, Tetrachlorethylen oder 1,1,1-Trichlorethan und ganz insbesondere dem Dichlormethan und dem Chloroform,
- oder aus Mischungen von diesen.

Das organische Lösungsmittel wird unter Beachtung der folgenden Voraussetzungen ausgewählt:
- es darf unter den gewählten Bedingungen so wenig wie möglich mit der wässrigen Lösung mischbar sein,
- es muss einen hohen Verteilungskoeffizienten für unerwünschte Furane aufweisen und damit ein gutes Lösungsmittel für die unerwünschten Furane sein,
- es muss einen höheren Verteilungskoeffizienten für die unerwünschten Furane aufweisen, als für 5-HMF, und die Differenz der Verteilungskoeffizienten von 5-HMF einerseits und von unerwünschten Furanen andererseits muss hinreichend groß sein, und
- es weist eine ausreichend hohe Differenz in seiner Dichte zu der wässrigen Lösung auf, sodass eine schnelle und effiziente Phasentrennung gefördert wird.

Auch Gemische, enthaltend halogenierte organische Lösungsmittel mit anderen organischen Lösungsmitteln können nützlich sein, solange das Gemisch die hierin für das Lösungsmittel beschriebenen Eigenschaften aufweist.

Nach einer weiteren vorteilhaften Ausgestaltung des Verfahrens ist das zweite organische Lösungsmittel in Schritt (B) ausgewählt aus
- den halogenierten organischen Lösungsmitteln, insbesondere Chloroform, Chlorbenzol, Trichlorethylen, Tetrachlorkohlenstoff, Fluorchlorkohlenstoffe, Dichlormethan, Tetrachlorethylen oder 1,1,1-Trichlorethan, weiter insbesondere den halogenierten Kohlenwasserstoffen Chloroform, Trichlorethylen, Tetrachlorkohlenstoff, Fluorchlorkohlenstoffe, Dichlormethan, Tetrachlorethylen oder 1,1,1-Trichlorethan und ganz insbesondere dem Dichlormethan und dem Chloroform,
- den Ketonen und insbesondere dem Methylisobutylketon,
- den aliphatischen Kohlenwasserstoffen und deren Derivaten, insbesondere dem 1-Butanol und dem 2-Butanol,
- den Furan-Derivaten und insbesondere dem 2-Methyltetrahydrofuran und dem Tetrahydrofuran,
- den alkylierten Phenolen, insbesondere dem o-Propylphenol und dem o-Isopropylphenol,
- oder aus Mischungen von diesen.

Das organische Lösungsmittel wird unter Beachtung der folgenden Voraussetzungen ausgewählt:
- es darf unter den gewählten Bedingungen so wenig wie möglich mit der wässrigen Lösung mischbar sein,
- es muss einen hohen Verteilungskoeffizienten für 5-HMF aufweisen und damit ein gutes Lösungsmittel für 5-HMF sein, und
- es weist eine ausreichend hohe Differenz in seiner Dichte zu der wässrigen Lösung auf, sodass eine schnelle und effiziente Phasentrennung gefördert wird.

Mischungen aus den organischen Lösungsmitteln sind ebenfalls gut geeignet, solange das Gemisch die hierin für das Lösungsmittel beschriebenen Eigenschaften aufweist, insbesondere zur Extraktion von 5-HMF dienen kann.

Nach einer weiteren bevorzugten Ausführungsform der Erfindung sind erstes und zweites organisches Lösungsmittel ausgewählt aus den halogenierten organischen Lösungsmitteln. Halogenierte organische Lösungsmittel erfüllen sowohl die an ein erstes organisches Lösungsmittel gestellten Anforderungen in Schritt (A), als auch die an ein zweites organisches Lösungsmittel gestellten Anforderungen in Schritt (B) des Verfahrens.

Nach einer weiteren vorteilhaften Ausgestaltung der Erfindung sind erstes und zweites organisches Lösungsmittel ausgewählt aus Dichlormethan und Mischungen enthaltend Dichlormethan. Die Verwendung von Dichlormethan als Lösungsmittel bringt weitere große Vorteile mit sich. So wird nach dem erfindungsgemäßen Verfahren 5-HMF in guter Ausbeute und hoher Reinheit gewonnen. Zusätzlich zu diesem vorteilhaften Ergebnis macht sich das erfindungsgemäße Verfahren die folgenden positiven Eigenschaften des Dichlormethans zu eigen: Es wurde gefunden, dass das Dichlormethan eine geringe Tendenz dazu aufweist, AbbauReaktionen von 5-HMF wie beispielsweise eine Polymerisation und damit eine Oligomerbildung zu katalysieren. Hierdurch ergibt sich 5-HMF mit einem besonders niedrigen Oligomergehalt. Zudem besitzt Dichlormethan eine geringe Mischbarkeit mit Wasser und eine vergleichsweise höhere Dichte, sodass sich zwei Phasen ausbilden, welche leicht trennbar sind. Dichlormethan ist zudem nichtentflammbar und minimiert lösungsmittelbedingte Risiken für Umwelt und Gesundheit. Von weiterem Vorteil ist ein relativ niedriger Siedepunkt (40°C), der neben einer energiesparenden, einfachen thermischen Entfernung von Dichlormethan zu einer Verminderung eines thermisch bedingten Abbaus von 5-HMF, insbesondere zu Oligomeren, führt.

Es war nicht zu erwarten, dass in Dichlormethan ein derart optimales Lösungsmittel zur Durchführung des erfindungsgemäßen Verfahrens gefunden werden könnte, da es insbesondere in Blumenthal et al. und den mit dem Fachartikel in Verbindung veröffentlichten Zusatzinformationen, die eine Vielzahl an geeigneten Lösungsmitteln vorschlagen, überhaupt nicht erwähnt wird. Ebenso verhält es sich mit Chloroform, das aufgrund seiner Eigenschaften ebenfalls bevorzugt ist.

Auch Gemische, enthaltend Dichlormethan und/oder Chloroform mit anderen organischen Lösungsmitteln können nützlich sein und sind daher ebenfalls bevorzugt.

Nach einer weiteren vorteilhaften Ausgestaltung des Verfahrens beträgt das Volumen des ersten organischen Lösungsmittels die 0,1 bis 0,7-fache Menge, bevorzugt die 0,1 bis 0,5-fache Menge des Volumens der wässrigen Lösung.

Nach einer weiteren vorteilhaften Ausgestaltung des Verfahrens beträgt das Volumen des zweiten organischen Lösungsmittels die 2,5 bis 7-fache Menge, bevorzugt die 4 bis 7-fache Menge des Volumens des Raffinats.

Nach einer weiteren vorteilhaften Ausgestaltung der Erfindung ist das polare Lösungsmittel Wasser, eine weitere wässrige Lösung oder das polare Lösungsmittel ist zumindest vollständig mit Wasser mischbar. Das polare Lösungsmittel kann 5-HMF enthalten. Bevorzugt ist die Konzentration an 5-HMF im polaren Lösungsmittel vor dem in Kontakt bringen mit der organischen Phase a2 geringer als die Konzentration an 5-HMF in der organischen Phase a2. Bevorzugt ist die Konzentration an 5-HMF im polaren Lösungsmittel vor dem in Kontakt bringen mit der organischen Phase a2 geringer als die Konzentration an 5-HMF in der wässrigen Lösung vor Schritt (A). Besonders bevorzugt ist das polare Lösungsmittel vor dem in Kontakt bringen mit der organischen Phase a2 frei von 5-HMF.

Nach einer weiteren vorteilhaften Ausgestaltung des Verfahrens werden die Schritte (A) und (B) bei Raumtemperatur durchgeführt. Hierdurch kann ein besonders schonendes Verfahren zur Gewinnung von 5-HMF zur Verfügung gestellt werden. Die Schritte können auch bei anderen Temperaturen betrieben werden, wobei die Grenzen nach unten durch die Viskositätszunahme in den Phasen und nach oben durch den Siedepunkt des organischen Lösungsmittels und den mit steigender Temperatur zunehmenden Abbau des 5-HMF zu Oligomeren gegeben sind.

Nach einer weiteren vorteilhaften Ausgestaltung der Erfindung umfasst das erfindungsgemäße Verfahren den folgenden weiteren Schritt
(C) partielles oder vollständiges Entfernen des in der organischen Phase b2 enthaltenen organischen Lösungsmittels.

Nach der Abtrennung der organischen Phase b2 in Schritt (B) ist es für vielfältige Anwendungen vorteilhaft, die mit 5-HMF beladene organische Phase b2 aufzukonzentrieren oder das 5-HMF von dem organischen Lösungsmittel zu isolieren. Insbesondere in der Vorbereitung eines Wechsels des Lösungsmittels ist dies vorteilhaft, da eine erhebliche Anzahl an Anwendungen polare oder wässrige Bedingungen erfordern. Eine vollständige Entfernung des Lösungsmittels, beispielsweise durch Trocknung oder Kristallisation, sowie eine partielle Entfernung sind dem Fachmann bekannt. Vorzugsweise wird das in der organischen Phase b2 enthaltene Lösungsmittel partiell oder vollständig durch Abdampfen entfernt. Bei dem partiellen Entfernen kann das Abdampfen bis zu einer gewünschten Konzentration an 5-HMF im Lösungsmittel geführt werden. Insbesondere vorteilhaft hierbei ist, dass sich eine optimal konzentrierte Lösungsmittel-5-HMF-Lösung einstellen lässt, die eine besonders geringe Tendenz von 5-HMF zur Oligomerbildung zeigt. Besonders bevorzugt wird das Abdampfen so geführt, dass die verbleibende Lösung ca. 15 Gew.% bis 40 Gew.% 5-HMF, bevorzugt 15 Gew.% bis 20 Gew.% 5-HMF aufweist.

Bevorzugt wird das Lösungsmittel beim Abdampfen zumindest teilweise mit einem Verdampfer entfernt, welcher eine kurze Verweilzeit des mit 5-HMF beladenen Lösungsmittels in der Eindampfungsstufe gestattet. Hierzu geeignet sind KurzwegVerdampfer und Fallfilm-Verdampfer. Bevorzugt sind Dünnschicht-Verdampfer. Die Verdampfer können auch in Kombination miteinander und/oder mit weiteren Einrichtungen zur Trennung von 5-HMF und Lösungsmittel, wie beispielsweise Rektifikationskolonnen, eingesetzt werden.

Besonders bevorzugt erfolgt das Abdampfen des Lösungsmittels bei unter 40°C, insbesondere bevorzugt erfolgt das Abdampfen bei 10°C bis 30°C und bei reduziertem Druck. Vorteilhafter ist eine Temperatur von 15°C bis 25°C und ein reduzierter Druck.

Nach einer weiteren vorteilhaften Ausgestaltung der Erfindung wird eingangs des erfindungsgemäßen Verfahrens eine Filtration der wässrigen Lösung vorgenommen. Beispielsweise können je nach Bedingungen der hydrothermalen Behandlung die in der wässrigen Lösung enthaltenen Verbindungen mehr oder weniger stark polymerisiert vorliegen. Zur Entfernung von in der wässrigen Lösung vorhandenen Partikeln wie Oligomere kann es dabei insbesondere sinnvoll sein, die wässrige Lösung eingangs des Verfahrens einer separaten Fest-Flüssig-Filtration zuzuführen. Hierzu wird beispielsweise eine Oberflächen-, Tiefenfiltration und/oder eine Membranfiltration durchgeführt. Auch kann eine Filtration dazu genutzt werden, mehr oder weniger große Anteile an löslichen Furan-Oligomeren aus der wässrigen Lösung, die mit dem erfindungsgemäßen Verfahren aufgearbeitet wird, zu entfernen.

Ein weiterer Gegenstand der Erfindung ist ein Verfahren zur Gewinnung von 5-HMF aus einer aus dem Herstellungsprozess von 5-HMF stammenden wässrigen Lösung gemäß dem unabhängigen Anspruch 10, nach dem die Schritte (A) und (B) in der Reihenfolge (B), (A) durchgeführt werden. Dabei wird in Schritt (B) anstelle des Raffinats die wässrige Lösung aus dem Herstellungesprozess in die Extraktionskolonne eingespeist, und das Verfahren umfasst nach Schritt (B) den folgenden weiteren Schritt (C) und einen Wechsel des Lösungsmittels. Die Durchführung der Schritte (A) und (B) des beschriebenen Verfahrens in der Reihenfolge (B), (A) hat zur Folge, dass nach Schritt (A) eine wässrige Phase erhalten wird, die das gereinigte 5-HMF enthält. Diese kann für verschiedene Zwecke sehr sinnvoll sein, insbesondere wenn das 5-HMF für nachfolgende Anwendungen in einer wässrigen Phase benötigt wird, wie beispielsweise einen wasser-basierten Oxidationsprozess. Die Durchführung der Schritte (A) und (B) in einer umgekehrten Reihenfolge hat zudem den Vorteil, dass sich hinsichtlich der Reinheit des erhaltenen 5-HMF und der Ausbeute keine Unterschiede ergeben. Die für die Umkehr der Schrittfolge benötigten Anpassungen der Verfahrensführung sind geringfügig und liegen im Können des Fachmanns.

Die vorstehend beschriebene Erfindung wird im Folgenden unter Bezugnahme auf die beigefügten Figuren näher erläutert.

Es zeigen:
Fig. 1 eine schematische Vergleichs-Darstellung einer Apparatur und eines Ablaufs einer Flüssig-Flüssig-Extraktion gemäß Schritt (B) im Gegenstrom mit einer Extraktorkolonne und mit Dichlormethan als organischem Lösungsmittel, ohne vorangeschaltetem Schritt (A).
Fig. 2 eine schematische Darstellung einer zur Durchführung des erfindungsgemäßen Verfahrens geeigneten Apparatur und des Ablaufs der Schritte (A) und (B) im Gegenstrom mit zwei Extraktionskolonnen und mit Dichlormethan als organischem Lösungsmittel.
Fig. 3 eine schematische Darstellung eines Verdampfers und der Aufkonzentrierung von mit 5-HMF beladenem Dichlormethan.

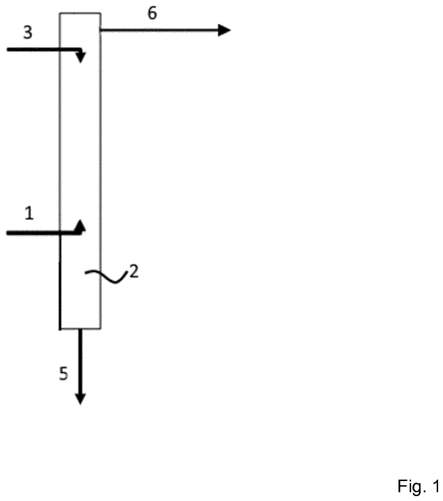

In Fig. 1 ist zum Vergleich schematisch der Ablauf einer Extraktion von 5-HMF aus einer verdünnten wässrigen Lösung, welche auf an sich bekannte Weise durch hydrothermale Behandlung von cellulosehaltiger Biomasse gewonnen wurde, mittels einer Flüssig-Flüssig-Extraktion dargestellt. Die Anteile der in der wässrigen Lösung enthaltenen Komponenten wie Furyl-Hydroxymethyl-Keton (FHK mit der IUPAC Bezeichnung 1-(2-Furyl)-2-hydroxyethanon), Furfural und lösliche Furan-Oligomere im Verhältnis zu 5-HMF können der Tabelle 1 (HMF-Lösung) entnommen werden. Die wässrige Lösung wird nach einer Filtration über die Zuleitung (1) im Bodenbereich einer Extraktionskolonne (2) eingespeist. Das Lösungsmittel Dichlormethan wird über die Zuleitung (3) am Kopf der Extraktionskolonne (2) eingeführt und bei Raumtemperatur im Gegenstrom zur wässrigen Lösung geleitet.

Das mit 5-HMF angereicherte Dichlormethan, welches über eine Ableitung (5) am Boden der Extraktionskolonne (2) als Extrakt entnommen wird, enthält ca. 7,5 Gew.% bis 10 Gew.% 5-HMF mit einer Reinheit von 88% bis 90%. Weitere, im Extrakt enthaltene substituierte Furane sind Furyl-Hydroxymethyl-Keton (FHK), Furfural und lösliche Furan-Oligomere. Die Anteile der im Extrakt enthaltenen Komponenten im Verhältnis zu 5-HMF können der Tabelle 1 (DCM-Extrakt 1) entnommen werden.

Das Raffinat, das die an 5-HMF verarmte wässrige Lösung bildet und welches den Hauptanteil an Nebenprodukten mit einer hohen Wasserlöslichkeit wie Lävulinsäure und andere Carbonsäuren sowie Zucker und Zuckerderivate enthält, wird über die Ableitung (6) aus der Extraktionskolonne (2) entfernt. Aus dem Raffinat können schließlich verschiedene Nebenprodukte gewonnen werden.

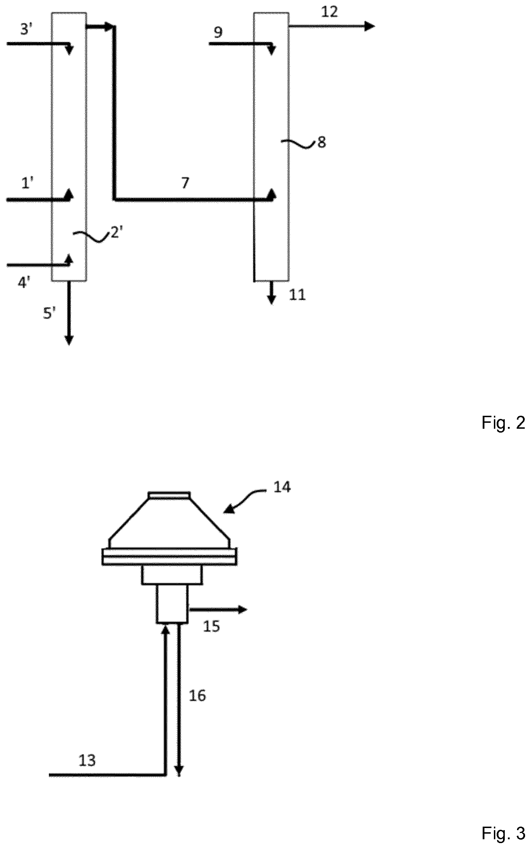

Fig. 2 zeigt schematisch den Ablauf einer Extraktion von 5-HMF den Flüssig-flüssig-Extraktionen gemäß Schritt (A) und (B) mit zwei hintereinander geschalteten Extraktionskolonnen aus der wässrigen Lösung, die zu der wässrigen Lösung aus Fig.1 identisch ist (Tabelle 1, HMF-Lösung). Die wässrige Lösung wird nach einer Filtration zunächst über die Zuleitung (1') im Bodenbereich einer ersten Extraktionskolonne (2') eingespeist, die invers (mit umgekehrter Dispergierrichtung) zur nachfolgenden betrieben wird, wobei die organische Phase dispers und die wässrige Phase kontinuierlich geführt wird. Das Lösungsmittel Dichlormethan wird über die Zuleitung (3') am Kopf der Extraktionskolonne (2') eingeführt und bei Raumtemperatur im Gegenstrom zur wässrigen Lösung geleitet.

Ein Rückstrom aus Wasser zur Rückextraktion von 5-HMF aus Dichlormethan wird der Extraktionskolonne (2') über die Zuleitung (4') zugeführt. Die Rückextraktion erfolgt durch Vermischen des beladenen Dichlormethans an einer Stelle stromabwärts - in Bezug auf die Strömungsrichtung des Dichlormethans - der Stelle, an der die wässrige Lösung in die Extraktionskolonne eingebracht wird. Das Wasser wird hierzu in die Extraktionskolonne an einer Stelle unterhalb des Zufuhrpunktes der wässrigen Lösung eingebracht und bildet einen zum Dichlormethan gegenläufigen Rückstrom.

Der Extraktionskolonne (2') wird über die Ableitung (5') mit FHK, Furfural und löslichen Furan-Oligomeren angereichertes Dichlormethan als ein erster Extrakt entnommen.

Ein erstes Raffinat, welches 5-HMF enthält, gelangt über die von dem Kopf der ersten Extraktionskolonne (2') ausgehende Leitung (7) in eine zweite Flüssig-flüssig-Extraktionskolonne (8), bei der es im Bodenbereich eingespeist wird. Die Flüssig-flüssig-Extraktionskolonne (8) wird mit derselben Dispergierrichtung geführt, wie die Extraktionskolonne (2) der Fig. 1, wobei die wässrige Phase als disperse und die organische Phase als kontinuierliche Phase geführt wird. Dichlormethan wird über die Zuleitung (9) am Kopf der zweiten Extraktionskolonne (8) eingeführt und bei Raumtemperatur im Gegenstrom zum ersten Raffinat geleitet.

Über eine Ableitung (11) am Boden der Extraktionskolonne (8) wird ein zweiter Extrakt entnommen, der ca. 7,5 Gew.% bis 10 Gew.% 5-HMF mit einer Reinheit von 97% bis 99% in Dichlormethan enthält. Die Anteile der im zweiten Extrakt enthaltenen Komponenten im Verhältnis zu 5-HMF können der Tabelle 1 (DCM-Extrakt 2) entnommen werden.

Das zweite Raffinat, welches den Hauptanteil an Nebenprodukten mit einer hohen Wasserlöslichkeit wie Lävulinsäure und andere Carbonsäuren sowie Zucker und Zuckerderivate enthält, wird über die Ableitung (12) aus der Extraktionskolonne (8) entfernt.

Fig. 3 zeigt schematisch die Aufkonzentrierung von mit 5-HMF beladenem Dichlormethan nach einer Abtrennung von der wässrigen Lösung. Das mit 5-HMF beladene Dichlormethan, das aus den Extraktionskolonnen (2) und (8), die in den Figuren 1 und 2 dargestellt sind, gewonnen wird, gelangt über die Leitung (13) in einen Dünnschicht-Verdampfer (14). Hierbei wird das aus einer Extraktionskolonne (2) oder (8) entnommene, mit 5-HMF beladene Lösungsmittel jeweils separat in den Dünnschicht-Verdampfer (14) eingeleitet, in welchem ein Teil des Dichlormethans verdampft, kondensiert und anschließend über die Ableitung (15) entfernt wird. Das Dichlormethan kann schließlich erneut den Extraktionskolonnen (2), (2') und/oder (8) zugeführt werden. Nach Eindampfung des aus der Extraktionskolonne (2) erhaltenen 5-HMF in Dichlormethan wird eine 5-HMF-Dichlormethan-Lösung mit ca. 15 Gew.% bis 20 Gew.% 5-HMF und einer Reinheit von 88% bis 90% erhalten. Nach Eindampfen des aus der Extraktionskolonne (8) erhaltenen 5-HMF in Dichlormethan wird eine 5-HMF-Dichlormethan-Lösung mit ca. 15 Gew.% bis 20 Gew.% 5-HMF und einer Reinheit von 97% bis 99% erhalten. Die aufkonzentrierten 5-HMF-Dichlormethan-Lösungen werden über die Ableitung (16) dem Dünnschicht-Verdampfer (14) entnommen.

**Tabelle 1**

| | **HMF-Lösung** | **DCM-Extrakt 1** | **DCM-Extrakt 2** |
|---|---|---|---|
| **Komponenten** | Soweit nicht anders Gew.% bezeichnet: alle relativ zu 5-HMF Angaben in | | |
| 5-HMF | (100) | (100) | (100) |
| Fructose | ca. 1 | <0,1 | <0,1 |
| Glucose und Zuckerintermediate | ca. 5 | <0,1 | <0,1 |
| Furyl-Hydroxymethyl-Keton (FHK) | 5 | ca. 5 | 0,5 - 1 |
| Furfural | 4 | ca. 4 | <0,1 |
| Lävulinsäure | 1 - 2 | <0,1 | <0,1 |
| Essigsäure | 0,5 - 1 | <0,1 | <0,1 |
| Ameisensäure | 0,5 - 1 | <0,1 | <0,1 |
| Formaldehyd | ca. 1 | <0,1 | <0,1 |
| Ionen (Nitrat, Natrium, etc.) | 0,25-0,3 | <10 ppm | <10 ppm |
| nicht näher untersuchte organische Nebenprodukte mit niedrigem Molekulargewicht | ca. 1 | <1 | <0,1 |
| lösliche Oligomere | ca. 2 | 1 -2 | <0,1 |

**Tabelle 2**

| | **DCM-Extrakt 1** | **DCM-Extrakt 2** |
|---|---|---|
| Reinheit HMF [%] | 88 bis 90 | 97 bis 99 |

In der Tabelle 2 ist die Reinheit des 5-HMF in den Extrakten 1 und 2 dargestellt. Insbesondere 5-HMF mit einer Reinheit von 97 bis 99 % eignet sich hervorragend als Plattformchemikalie für eine Reihe industrieller Anwendungen und dient beispielsweise als Vorstufe für die Synthese verschiedener nicht-mineralölbasierter Lösungsmittel, Tenside, Pharmazeutika, Pflanzenschutzmittel und Verbindungen zur Herstellung von Polymeren. 2,5-Furandicarbonsäure und 2,5-Diformylfuran sind derartige Verbindungen, die beispielsweise durch Oxidation aus dem 5-HMF erhalten werden können.

Weitere Vorteile und vorteilhafte Ausgestaltungen sind den Ansprüchen zu entnehmen. Sämtliche Merkmale der Erfindung können sowohl einzeln als auch in beliebiger Kombination miteinander erfindungswesentlich sein.

### BEZUGSZEICHENLISTE

- 1, 1': Zuleitung
- 2: Extraktionskolonne
- 2': erste Extraktionskolonne
- 3, 3': Zuleitung
- 4': Wasch-Rückstrom
- 5, 5': Ableitung
- 6: Ableitung
- 7: Leitung
- 8: zweite Extraktionskolonne
- 9: Zuleitung
- 10 11: Ableitung
- 12: Ableitung
- 13: Leitung
- 14: Dünnschicht-Verdampfer
- 15: Ableitung
- 16: Ableitung

## Patentansprüche

1. Verfahren zur Gewinnung von 5-HMF aus einer aus dem Herstellungsprozess von 5-HMF stammenden wässrigen Lösung umfassend die Schritte:
(A) Kontinuierliche flüssig-flüssig-Extraktion, bei der
die wässrige Lösung in einer Extraktionskolonne im Gegenstrom mit einem ersten organischen Lösungsmittel, dessen Volumen höchstens die 0,7-fache Menge des Volumens der wässrigen Lösung beträgt, unter Ausbildung einer wässrigen Raffinatphase a1 und einer organischen Phase a2 so in Kontakt gebracht wird, dass in einem ersten Abschnitt der Extraktionskolonne die wässrige Raffinatphase a1 als kontinuierliche Phase und die organische Phase a2 als disperse Phase geführt wird, und dass sich die disperse organische Phase a2 in einem zweiten Abschnitt der Extraktionskolonne vereinigt und eine kontinuierliche organische Phase a2 bildet, und
die kontinuierliche organische Phase a2 in dem zweiten Abschnitt mit einem polaren Lösungsmittel in Kontakt gebracht wird, welches die Extraktionskolonne im Gegenstrom zu dem organischen Lösungsmittel durchströmt, wobei sich das polare Lösungsmittel in dem ersten Abschnitt mit der wässrigen Raffinatphase a1 vereinigt, und
die resultierende Raffinatphase a1 der Extraktionskolonne als Raffinat entnommen wird,
(B) Kontinuierliche flüssig-flüssig-Extraktion bei der
das Raffinat in einer Extraktionskolonne im Gegenstrom mit einem zweiten organischen Lösungsmittel, dessen Volumen mindestens die 2,5-fache Menge des Volumens des Raffinats beträgt, unter Ausbildung einer wässrigen Raffinatphase b1 und einer organischen Phase b2 so in Kontakt gebracht wird, dass in einem Abschnitt der Extraktionskolonne die wässrige Raffinatphase b1 als disperse Phase und die organische Phase b2 als kontinuierliche Phase geführt wird, und
die organische Phase b2 der Extraktionskolonne als Extrakt entnommen wird.

2. Verfahren nach Anspruch 1, **dadurch gekennzeichnet, dass** das erste organische Lösungsmittel in Schritt (A) ausgewählt ist aus
- den halogenierten organischen Lösungsmitteln, insbesondere Chloroform, Chlorbenzol, Trichlorethylen, Tetrachlorkohlenstoff, Fluorchlorkohlenstoffe, Dichlormethan, Tetrachlorethylen oder 1,1,1-Trichlorethan, weiter insbesondere den halogenierten Kohlenwasserstoffen Chloroform, Trichlorethylen, Tetrachlorkohlenstoff, Fluorchlorkohlenstoffe, Dichlormethan, Tetrachlorethylen oder 1,1,1-Trichlorethan und ganz insbesondere dem Dichlormethan und dem Chloroform,
- oder aus Mischungen von diesen.

3. Verfahren nach Anspruch 1 oder 2, **dadurch gekennzeichnet, dass** das zweite organische Lösungsmittel in Schritt (B) ausgewählt ist aus
- den halogenierten organischen Lösungsmitteln, insbesondere Chloroform, Chlorbenzol, Trichlorethylen, Tetrachlorkohlenstoff, Fluorchlorkohlenstoffe, Dichlormethan, Tetrachlorethylen oder 1,1,1-Trichlorethan, weiter insbesondere den halogenierten Kohlenwasserstoffen Chloroform, Trichlorethylen, Tetrachlorkohlenstoff, Fluorchlorkohlenstoffe, Dichlormethan, Tetrachlorethylen oder 1,1,1-Trichlorethan und ganz insbesondere dem Dichlormethan und dem Chloroform,
- den Ketonen und insbesondere dem Methylisobutylketon,
- den aliphatischen Kohlenwasserstoffen und deren Derivaten, insbesondere dem 1-Butanol und dem 2-Butanol,
- den Furan-Derivaten und insbesondere dem 2-Methyltetrahydrofuran und dem Tetrahydrofuran,
- den alkylierten Phenolen, insbesondere dem o-Propylphenol und dem o-Isopropylphenol,
- oder aus Mischungen von diesen.

4. Verfahren nach Anspruch 1, **dadurch gekennzeichnet, dass** erstes und zweites organisches Lösungsmittel ausgewählt sind aus den halogenierten organischen Lösungsmitteln.

5. Verfahren nach Anspruch 1, **dadurch gekennzeichnet, dass** erstes und zweites organisches Lösungsmittel ausgewählt sind aus Dichlormethan und Mischungen enthaltend Dichlormethan.

6. Verfahren nach einem der vorhergehenden Ansprüche, **dadurch gekennzeichnet, dass** das polare Lösungsmittel Wasser ist.

7. Verfahren nach einem der vorhergehenden Ansprüche, **dadurch gekennzeichnet, dass** das Volumen des ersten organischen Lösungsmittels die 0,1 bis 0,7-fache Menge, bevorzugt die 0,1 bis 0,5-fache Menge des Volumens der wässrigen Lösung beträgt.

8. Verfahren nach einem der vorhergehenden Ansprüche, **dadurch gekennzeichnet, dass** das Volumen des zweiten organischen Lösungsmittels die 2,5 bis 7-fache Menge, bevorzugt die 4 bis 7-fache Menge des Volumens des Raffinats beträgt.

9. Verfahren nach einem der vorhergehenden Ansprüche, **dadurch gekennzeichnet, dass** das erfindungsgemäße Verfahren den folgenden weiteren Schritt umfasst
(C) partielles oder vollständiges Entfernen des in der organischen Phase b2 enthaltenen organischen Lösungsmittels.

10. Verfahren zur Gewinnung von 5-HMF aus einer aus dem Herstellungsprozess von 5-HMF stammenden wässrigen Lösung umfassend die Schritte (A) und (B) gemäß einem der Ansprüche 1 bis 8 in der Reihenfolge (B), (A),
wobei in Schritt (B) anstelle des Raffinats die wässrige Lösung aus dem Herstellungsprozess in die Extraktionskolonne eingespeist wird,
wobei das Verfahren nach Schritt (B) den folgenden weiteren Schritt
(C) partielles oder vollständiges Entfernen des in der organischen Phase b2 enthaltenen organischen Lösungsmittels
und einen Wechsel des Lösungsmittels umfasst.

11. Verfahren nach einem der vorhergehenden Ansprüche, **dadurch gekennzeichnet, dass** eingangs des erfindungsgemäßen Verfahrens eine Filtration der wässrigen Lösung vorgenommen wird.

## Claims

1. Method for obtaining 5-HMF from an aqueous solution that results from the production process of 5-HMF, comprising the steps of:
(A) continuous liquid-liquid extraction in which the aqueous solution, in an extraction column, is countercurrently brought into contact with a first organic solvent, the volume of which is at most 0.7 times the volume of the aqueous solution, thereby forming an aqueous raffinate phase a1 and an organic phase a2, in such a way that the aqueous raffinate phase a1 is guided as a continuous phase and the organic phase a2 is guided as a disperse phase in a first portion of the extraction column, and in such a way that the disperse organic phase a2 combines and forms a continuous organic phase a2 in a second portion of the extraction column, and the continuous organic phase a2 is brought into contact in the second portion with a polar solvent which flows through the extraction column countercurrently to the organic solvent, wherein the polar solvent combines with the aqueous raffinate phase a1 in the first portion, and
the resulting raffinate phase a1 is extracted from the extraction column as a raffinate,
(B) continuous liquid-liquid extraction in which the raffinate, in an extraction column, is countercurrently brought into contact with a second organic solvent, the volume of which is at least 2.5 times the volume of the raffinate, thereby forming an aqueous raffinate phase b1 and an organic phase b2, in such a way that the aqueous raffinate phase b1 is guided as a disperse phase and the organic phase b2 is guided as a continuous phase in a portion of the extraction column, and
the organic phase b2 is extracted from the extraction column as an extract.

2. Method according to claim 1, **characterized in that** the first organic solvent in step (A) is selected from
- the halogenated organic solvents, in particular chloroform, chlorobenzene, trichloroethylene, carbon tetrachloride, chlorofluorocarbons, dichloromethane, tetrachloroethylene or 1,1,1-trichloroethane, more particularly the halogenated hydrocarbons chloroform, trichloroethylene, carbon tetrachloride, chlorofluorocarbons, dichloromethane, tetrachloroethylene or 1,1,1-trichloroethane, and most particularly dichloromethane and chloroform,
- or mixtures thereof.

3. Method according to either claim 1 or 2, **characterized in that** the second organic solvent in step (B) is selected from
- the halogenated organic solvents, in particular chloroform, chlorobenzene, trichloroethylene, carbon tetrachloride, chlorofluorocarbons, dichloromethane, tetrachloroethylene or 1,1,1-trichloroethane, more particularly the halogenated hydrocarbons chloroform, trichloroethylene, carbon tetrachloride, chlorofluorocarbons, dichloromethane, tetrachloroethylene or 1,1,1-trichloroethane, and most particularly dichloromethane and chloroform,
- the ketones and in particular methyl isobutyl ketone,
- the aliphatic hydrocarbons and their derivatives, in particular 1-butanol and 2-butanol,
- the furan derivatives and in particular 2-methyltetrahydrofuran and tetrahydrofuran,
- the alkylated phenols, in particular o-propylphenol and o-isopropylphenol,
- or mixtures thereof.

4. Method according to claim 1, **characterized in that** the first and second organic solvents are selected from the halogenated organic solvents.

5. Method according to claim 1, **characterized in that** the first and second organic solvents are selected from dichloromethane and mixtures containing dichloromethane.

6. Method according to any of the preceding claims, **characterized in that** the polar solvent is water.

7. Method according to any of the preceding claims, **characterized in that** the volume of the first organic solvent is 0.1 to 0.7 times, preferably 0.1 to 0.5 times the volume of the aqueous solution.

8. Method according to any of the preceding claims, **characterized in that** the volume of the second organic solvent is 2.5 to 7 times, preferably 4 to 7 times the volume of the raffinate.

9. Method according to any of the preceding claims, **characterized in that** the method according to the invention comprises the following additional step (C) partial or complete removal of the organic solvent contained in the organic phase b2.

10. Method for obtaining 5-HMF from an aqueous solution that results from the production process of 5-HMF, comprising steps (A) and (B) according to any of claims 1 to 8 in the order (B), (A), wherein, instead of the raffinate, the aqueous solution from the production process is fed into the extraction column in step (B), wherein the method comprises, after step (B), the following additional step
(C) partial or complete removal of the organic solvent contained in the organic phase b2
and changing the solvent.

11. Method according to any of the preceding claims, **characterized in that** the aqueous solution is filtered at the beginning of the method according to the invention.

## Revendications

1. Procédé d'obtention de 5-HMF à partir d'une solution aqueuse issue du procédé de production de 5-HMF, comprenant les étapes :
(A) d'extraction liquide-liquide en continu, dans laquelle la solution aqueuse dans une colonne d'extraction à contre-courant est mise en contact avec un premier solvant organique, dont le volume est au plus 0,7 fois le volume de la solution aqueuse, avec formation d'une phase aqueuse de raffinat a1 et d'une phase organique a2, de sorte que, dans une première section de la colonne d'extraction, la phase aqueuse de raffinat a1 est conduite en phase continue et la phase organique a2 est conduite en phase dispersée, et que la phase organique dispersée a2 est combinée dans une seconde section de la colonne d'extraction et forme une phase organique continue a2, et
la phase organique continue a2 est mise en contact dans la seconde section avec un solvant polaire qui traverse la colonne d'extraction à contre-courant du solvant organique, le solvant polaire se combinant dans la première section avec la phase aqueuse de raffinat a1, et
la phase de raffinat résultante a1 étant éliminée de la colonne d'extraction sous forme de raffinat,
(B) d'extraction liquide-liquide en continu, dans laquelle le raffinat dans une colonne d'extraction à contre-courant est mis en contact avec un second solvant organique dont le volume est au moins 2,5 fois le volume du raffinat, avec formation d'une phase aqueuse de raffinat b1 et d'une phase organique b2 de sorte que, dans une section de la colonne d'extraction, la phase aqueuse de raffinat b1 est conduite en phase dispersée et la phase organique b2 est conduite en phase continue, et
la phase organique b2 est éliminée de la colonne d'extraction sous forme d'extrait.

2. Procédé selon la revendication 1, **caractérisé en ce que** le premier solvant organique de l'étape (A) est choisi parmi
- les solvants organiques halogénés, en particulier le chloroforme, le chlorobenzène, le trichloréthylène, le tétrachlorure de carbone, les chlorofluorocarbures, le dichlorométhane, le tétrachloroéthylène ou le 1,1,1-trichloroéthane, en outre en particulier les hydrocarbures halogénés chloroforme, trichloréthylène, tétrachlorure de carbone, chlorofluorocarbures, dichlorométhane, tétrachloroéthylène ou 1,1,1-trichloroéthane et tout particulièrement le dichlorométhane et le chloroforme,
- ou des mélanges de ceux-ci.

3. Procédé selon la revendication 1 ou 2, **caractérisé en ce que** le second solvant organique de l'étape (B) est choisi parmi
- les solvants organiques halogénés, en particulier le chloroforme, le chlorobenzène, le trichloréthylène, le tétrachlorure de carbone, les chlorofluorocarbures, le dichlorométhane, le tétrachloroéthylène ou le 1,1,1-trichloroéthane, en outre en particulier les hydrocarbures halogénés chloroforme, trichloréthylène, tétrachlorure de carbone, chlorofluorocarbures, dichlorométhane, tétrachloroéthylène ou 1,1,1-trichloroéthane et tout particulièrement le dichlorométhane et le chloroforme,
- les cétones et en particulier la méthylisobutylcétone,
- les hydrocarbures aliphatiques et leurs dérivés, en particulier le 1-butanol et le 2-butanol,
- les dérivés furaniques et en particulier le 2-méthyltétrahydrofurane et le tétrahydrofurane,
- les phénols alkylés, notamment l'o-propylphénol et l'o-isopropylphénol,
- ou des mélanges de ceux-ci.

4. Procédé selon la revendication 1, **caractérisé en ce que** les premier et second solvants organiques sont choisis parmi les solvants organiques halogénés.

5. Procédé selon la revendication 1, **caractérisé en ce que** les premier et second solvants organiques sont choisis parmi le dichlorométhane et les mélanges contenant du dichlorométhane.

6. Procédé selon l'une des revendications précédentes, **caractérisé en ce que** le solvant polaire est l'eau.

7. Procédé selon l'une des revendications précédentes, **caractérisé en ce que** le volume du premier solvant organique est de 0,1 à 0,7 fois la quantité, de préférence de 0,1 à 0,5 fois la quantité du volume de la solution aqueuse.

8. Procédé selon l'une des revendications précédentes, **caractérisé en ce que** le volume du second solvant organique est de 2,5 à 7 fois la quantité, de préférence de 4 à 7 fois la quantité du volume du raffinat.

9. Procédé selon l'une des revendications précédentes, **caractérisé en ce que** le procédé selon l'invention comprend l'étape supplémentaire suivante :
(C) l'élimination partielle ou totale du solvant organique contenu dans la phase organique b2.

10. Procédé d'obtention de 5-HMF à partir d'une solution aqueuse provenant du procédé de production de 5-HMF comprenant les étapes (A) et (B) selon l'une des revendications 1 à 8 dans l'ordre (B), (A),
dans lequel, à l'étape (B) au lieu du raffinat, la solution aqueuse issue du processus de production est introduite dans la colonne d'extraction,
dans lequel le procédé, après l'étape (B), comprend l'étape supplémentaire suivante :
(C) l'élimination partielle ou totale du solvant organique contenu dans la phase organique b2
et un changement du solvant.

11. Procédé selon l'une des revendications précédentes, **caractérisé en ce que**, au début du procédé selon l'invention, un filtrage de la solution aqueuse est effectué.
